# EUROPEAN PATENT APPLICATION

(11) **EP 4 129 399 A1**
(43) Date of publication of application: **08.02.2023**
(21) Application number: 21189349.0
(22) Date of filing: 03.08.2021
(51) Int. Cl.: A61N 5/10

(54) **MOTION REDUCTION IN DIAGNOSTIC IMAGING OR RADIATION THERAPY**

(71) Applicant: Koninklijke Philips N.V., 5656 AG Eindhoven (NL)
(72) Inventor: LEUSSLER, Christoph Günther, 5656 AE Eindhoven (NL); WEISS, Steffen, 5656 AE Eindhoven (NL)
(74) Representative: Philips Intellectual Property & Standards

(57) **Abstract**

The present invention relates to monitoring motion in diagnostic imaging or radiation therapy. In order to improve workflow, a device is proposed that comprises an input unit, a processing unit, and an output unit. The input unit is configured to receive an electroencephalogram (EEG) signal measured from a patient. The processing unit is configured to determine a readiness potential (RP) based on the received EEG signal, to determine whether patient motion is likely to happen based on the received EEG signal, and to provide a control signal via the output unit as output, if it is predicted that patient motion is likely to happen. The control signal is usable for controlling an apparatus to perform a function to reduce motion artefacts during the medical imaging or to assure that radiation dose is delivered according to a planned dose map during the radiation therapy

## Description

### FIELD OF THE INVENTION

The present invention relates to monitoring motion in diagnostic imaging or radiation therapy. In particular, the present invention relates to a device, a system, and a computer-implemented method for monitoring motion during medical imaging or during radiation therapy, and to a computer program product.

### BACKGROUND OF THE INVENTION

For hospitals, a high patient throughput in medical imaging is crucial as well as good image quality at the same time. Therefore, it is very important that the patient supports the imaging procedure as much as possible. This leads to the need of good supporting tools to inform and guide the patient but also to show how good he/she is executing the requested actions, especially in an autonomous image acquisition scenario.

Motion is the most prominent source of image artefacts, scan aborts or scan repetitions in medical imaging although a large variety of approaches are actually used to minimize motion artefacts. Most approaches use special acquisition methods to minimize artefacts. The commonly known approaches to avoid motion are immobilization of the patient.

### SUMMARY OF THE INVENTION

There may be a need to improve workflow.

The object of the present invention is solved by the subject-matter of the independent claims. Further embodiments and advantages of the invention are incorporated in the dependent claims. Furthermore, it shall be noted that all embodiments of the present invention concerning a computer-implemented method might be carried out with the order of the steps as described, nevertheless this has not to be the only and essential order of the steps of the method as presented herein. The computer-implemented method disclosed herein can be carried out with another order of the disclosed steps without departing from the respective method embodiment, unless explicitly mentioned to the contrary hereinafter.

Technical terms are used by their common sense. If a specific meaning is conveyed to certain terms, definitions of terms will be given in the following in the context of which the terms are used.

According to a first aspect of the present invention, there is provided a device for monitoring motion during medical imaging or during radiation therapy. The device comprises an input unit, a processing unit, and an output unit. The input unit is configured to receive an electroencephalogram (EEG) signal measured from a patient. The processing unit is configured to determine a readiness potential (RP) based on the received EEG signal, to determine whether patient motion is likely to happen based on the received EEG signal, and

To provide a control signal via the output unit as output, if it is predicted that patient motion is likely to happen. The control signal is usable for controlling an apparatus to perform a function to reduce motion artefacts during the medical imaging or to assure that radiation dose is delivered according to a planned dose map during the radiation therapy.

In other words, it is proposed to detect early signals of decision making of the patient with the aid of an EEG sensor. The EEG sensor measures the preparatory signals in form of EEG data and the apparatus receives and processes these to predict upcoming movements of the patient. If it is predicted that patient motion is likely to happen, one or more control signals may be generated to control one or more apparatuses to perform one or more of the following counter-measures:
- to intervene and prevent motion with the help of the patient. To prevent the motion, the imaging system may be equipped with means to inform the patient about an upcoming motion event as already recognized by the EEG sensor. Before the imaging session, the patient may have been trained to suppress the motion upon this veto signal.
- to prevent motion mechanically. Here, mechanical means, such as immobilization devices or even counteractive actuators, may be used to limit or counteract the motion as it is performed by the patient.
- to adapt the image acquisition process in order to account for motion that will happen in the next moment. A diagnostic imaging system may be equipped with methods to minimize the effect of such expected motion on image quality if warned by the proposed apparatus before the onset of the motion.
- to adapt the radiation delivery process in order to account for motion that will happen in the next moment. In an example, it is possible to mechanically stop the patient motion. In another example, it is possible to redirect therapy if this is more appropriate or convenient than stopping the upcoming patient motion mechanically.

According to an embodiment of the present invention, the processing unit is configured to apply a pre-trained machine-learning algorithm to derive the RP from the received EEG signal.

The pre-trained machine-learning algorithm is at least one of an artificial neural network, a support vector machine, a Bayesian network, a decision tree, a linear discriminant and a nearest-neighbour classifier.

According to an embodiment of the present invention, the input unit is configured to receive at least one physiological signal different from the EEG signal. The processing unit is configured to determine whether patient motion is likely to happen based on the received EEG signal and the at least one physiological signal.

For example, a magnetoencephalograph (MEG), an electromyography (EMG), an electrocardiograph (ECG/EKG), a camera, a range imaging camera, a thermal probe or camera, a pressure sensor, or any sensor measuring any physiological state indicative of any upcoming motion, or any combination thereof, may be used to provide the at least one physiological signal.

According to an embodiment of the present invention, the control signal comprises a signal configured to control an informing device to inform the patient that patient motion is likely to happen such that the patient can proactively react.

Examples of informing device may include, but are not limited to, an optical interface (e.g., LED, Monitor), a mechanical actuator integrated into radiofrequency (RF) coils or matrass, a haptic feedback device, and an actuator in a glove.

According to an embodiment of the present invention, the control signal comprises a signal configured to control a robot and/or an actuator to provide a counter-measure such that a counter force is applied to compensate for the patient motion.

In other words, mechanical means, such as immobilization devices or even counteractive actuators, may be used to limit or counteract the motion as it is likely to be performed by the patient.

For example, an actuator may provide a mechanical counter force to prevent or reduce motion.
For example, a head support, a patient fixation, and/or a part of the patient support may be activated to immobilize the parts of the patient to prevent or minimize motion.

According to an embodiment of the present invention, the actuator is located in one or more of the followings: in a head-fixation holder, in a body-fixation holder, in a head coil, and in a mattress.

According to an embodiment of the present invention, the control signal comprises a signal configured to control an imaging device to modify a medical imaging acquisition process in order to account for the patient motion that is likely to happen.

In other words, the acquisition process may be adapted in order to account for motion that will happen in the next moment. The diagnostic system is therefore equipped with methods to minimize the effect of such expected motion on image quality if warned by the EEG sensor before the onset of the motion.

Taking MR imaging as an example, it is possible to adapt the k-space acquisition to account for motion that is about to happen. In another example, it is possible to adapt the MR sequence in time domain to account for motion that is about to happen.

According to an embodiment of the present invention, the control signal comprises a signal configured to control a therapy device to modify a therapy process in order to account for the patient motion that is likely to happen; and
wherein the modification of the therapy process comprises one of:
- ceasing the therapy process; and
- redirecting therapy delivery.

According to an embodiment of the present invention, the control signal comprises a signal configured to activate a motion detector to detect whether the patient is moving.

Examples of the motion detector may include, but are not limited to, 3D sensing device (e.g., RADAR, LIDAR, or LASER), a camera, etc.

According to a second aspect of the present invention, there is provided a system, which comprises a monitoring system configured to measuring an electroencephalogram (EEG) signal of a patient, and a device according to the first aspect and any associated example configured to receive the measured EEG signal and to provide a control signal as output, if it is predicted that patient motion is likely to happen.

An example of the monitoring system is a computer brain interface.
According to an embodiment of the present invention, the system comprises one or more of:
- an informing device configured to inform the patient that patient motion is likely to happen, in response to the control signal;
- a robot and/or an actuator to provide a counter-measure such that a counter force is applied to compensate for the patient motion in response to the control signal; and
- a motion detector configured to be activated to detect whether the patient is moving, in response to the control signal.

According to an embodiment of the present invention, the system is a medical imaging system that comprises an imaging device configured to acquire image data of the patient. The imaging device is configured to modify a medical imaging acquisition process in order to account for the patient motion that is likely to happen, in response to the control signal.

According to an embodiment of the present invention, the system is a radiation therapy system comprising a therapy device configured to deliver ionizing radiation. The therapy device is configured to modify a therapy process in order to account for the patient motion that is likely to happen, in response to the control signal.
According to a third aspect of the present invention, there is provided a computer-implemented method for monitoring motion during medical imaging or during radiation therapy, comprising:
- receiving an electroencephalogram (EEG) signal measured from a patient;
- determining a readiness potential (RP) based on the received EEG signal;
- determining whether patient motion is likely to happen based on the received EEG signal; and
- providing a control signal via the output unit as output, if it is predicted that patient motion is likely to happen;
wherein the control signal is usable for controlling an apparatus to perform a function to reduce motion artefacts during the medical imaging or to assure that radiation dose is delivered according to a planned dose map during the radiation therapy.

According to a fourth aspect of the present invention, there is provided a computer program product comprising instructions which, when the program is executed by at least one processing unit, cause the at least one processing unit to carry out the steps of the method according to the third aspect of the present invention and any associated example.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 shows a block diagram of an exemplary device for monitoring motion during medical imaging or during radiation therapy
Fig. 2 shows an example of a system.
Fig. 3 shows another example of a system.
Fig. 4 shows a flow chart describing a computer-implemented method for monitoring motion during medical imaging or during radiation therapy.
Fig. 5 shows an overview of exemplary control signals generated based on the EEG data.
Fig. 6 shows an exemplary feedback-reaction loop-model for implementing the method.
Fig. 7 shows an example of a patient learning and guidance tool.

### DETAILED DESCRIPTION OF EMBODIMENTS

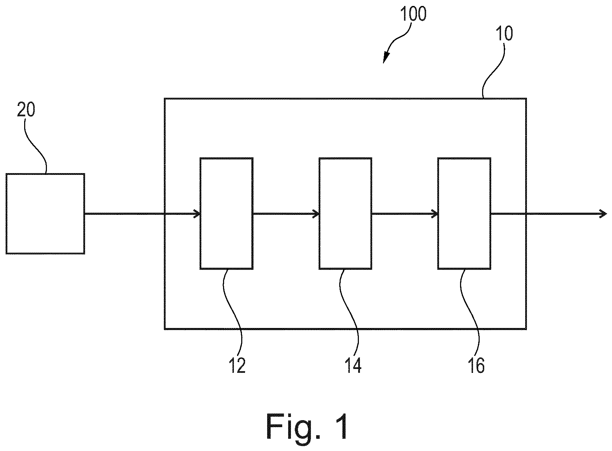

Fig. 1 illustrates a block diagram of an exemplary device 10 for monitoring motion during medical imaging or during radiation therapy, in accordance with an embodiment. The device 10 may receive a measured EEG signal from a monitoring system 20. The device 10 and the monitoring system 20 together form a system 100.

The device 10 includes an input unit 12, a processing unit 14, and an output unit 16.

In general, the device 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 1 may show a limited number of components by way of example, it can be appreciated that a greater or a fewer number of components may be employed for a given implementation.

In some examples, the device 10 may be implemented by a computing platform such as a mobile platform, personal computer (PC) platform, and/or consumer electronics (CE) platform supporting various networking, communications, and/or multimedia capabilities. Such capabilities may be supported by various networks, such as a Wide Area Network (WAN), Local Area Network (LAN), Metropolitan Area Network (MAN), wireless WAN (WWAN), wireless LAN (WLAN), wireless MAN (WMAN), wireless personal area network (WPAN), Worldwide Interoperability for Microwave Access (WiMAX) network, broadband wireless access (BWA) network, the Internet, and/or any other wired or wireless network in accordance with the described embodiments.

In some implementations, the device 10 may comprise a system within and/or coupled to a computing device such as PC, desktop PC, notebook PC, laptop computer, mobile internet device (MID), mobile computing device, smart phone, personal digital assistant (PDA), mobile telephone, or other type of computing device in accordance with the described embodiments. The computing device may include, for example, an electronic display.

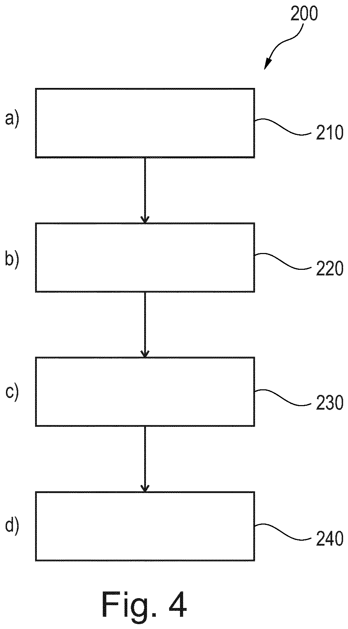

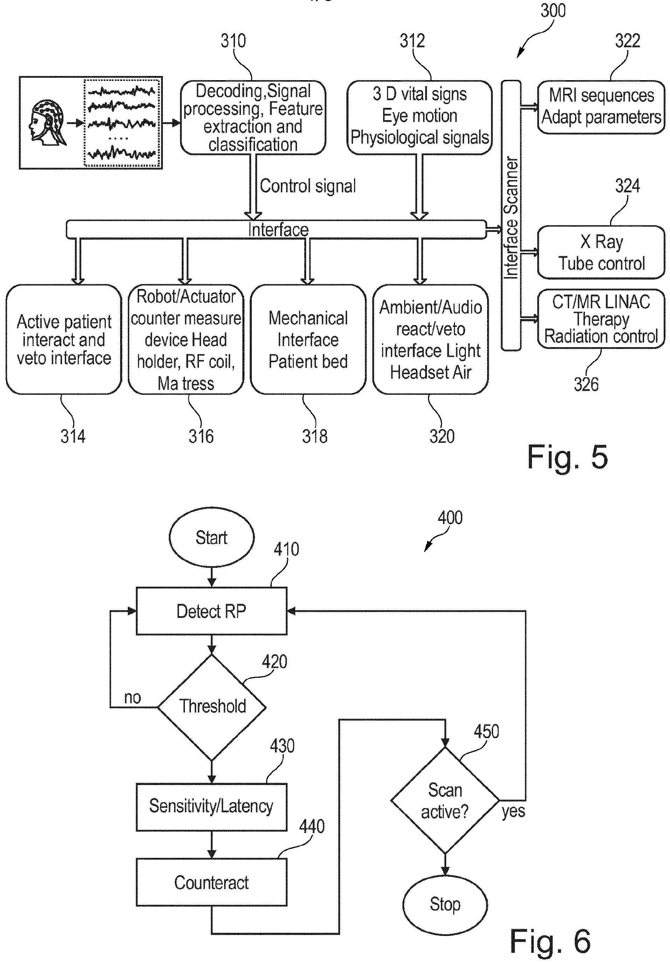

The processing unit 12 may execute instructions to perform the method described herein, which will be explained in detail with respect to the embodiments shown in Figs. 4 and 5. Optionally, the device 10 may comprise a memory (not shown), which may include, but is not limited to, volatile memory and/or non-volatile memory. The memory may be used to store processor instructions, and other data and instructions to enable the processing unit 12 to perform the techniques described herein.

The input unit 12 and the output unit 16 may include hardware and/or software to enable the device 10 to receive data from the monitoring system 20, and to communicate with other devices and/or a network. For example, the input unit 12 may receive data via a wired connection or via a wireless connection. The input unit 12 and the output unit 16 may also provide cellular telephone communications, and/or other data communications for the device 10.

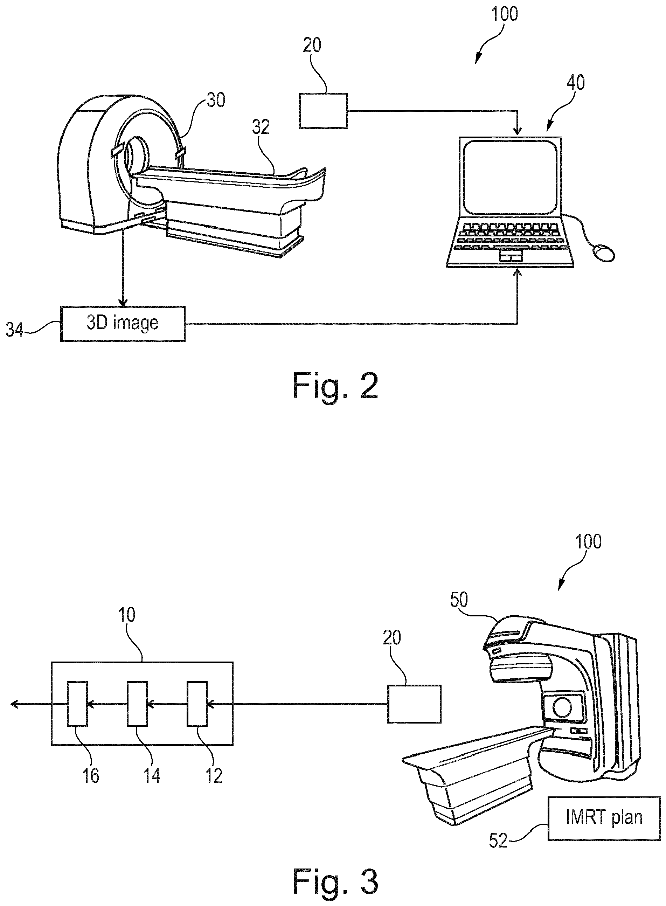

Fig. 2 illustrates an example of a system 100, in accordance with an embodiment of the present disclosure. In this example, the system is a medical imaging system, which comprises a monitoring system 20, an imaging device 30, and a computer workstation 40.

The imaging device 30 may include e.g., a magnetic resonance (MR) imaging scanner, a computer tomography (CT) imaging device, or so forth. In the example of Fig. 2 an MR imaging system is shown, which typically includes a magnet to impose the static magnetic field, gradient coils for imposing spatially distributed gradient magnetic fields along three orthogonal coordinates, and RF coils to transmit and receive RF signals to and from the selected nuclei of the body being imaged. A patient may lie on a patient table 32 such that a portion of the patient to be imaged is moved, in three-dimensions, into an "imaging volume" between the magnet and coils, which defines a field of view (FOV) of the MRI system.

The MRI system operator controls the system through the computer workstation 40 with a keyboard, screen and other operator input/output devices. The MRI system operator positions the patient within the imaging volume using a movable table 32, and selects one or more imaging parameters, such as: (a) imaging technique, e.g., diagnostic MRI, fast-MRI, MR fluoroscopy, and MR vascular imaging; (b) pulse sequence, e.g., spin echo, field echo, inversion recovery, fast spin echo and fast field echo; (c) imaging modes, e.g., multi-slice MR scans, multi-slab three-dimensional (3D) scans, multi-echo scans, multi-coverage (to cover an area greater than that covered by a single scan), and multi-angle acquisition (multiple groups of slices with different angles in the same TR); (d) fat suppression and separation techniques, and (e) artifact suppression techniques.

After the desired imaging parameters have been selected, the MRI system is programmed to scan the patient with one or more respectively corresponding pulse sequence(s) of RF pulses, sliceselection, phase encoding magnetic gradient pulses, and read-out magnetic gradient field pulses. When the diagnostic scan is initiated, a predetermined pulse-sequence is repeated to generate a series of nuclear magnetic resonance (NMR) RF responsive signals from the excited nuclei of the patient. The MRI system analyzes these signals and generates images 34 of the internal organs and tissues of the patient based on the responsive RF signals.

The monitoring system 20 may include any sensor that is capable of detecting an EEG signal of the patient during the imaging process. In some examples, the monitoring system 20 may be a computer brain interface (CBI) with an EEG sensor.

In the example of Fig. 2, the EEG signal measured by the monitoring system 20 is provided to the computer workstation 40, in which the device 10 may be resident running as software routines.

As will be explained in detail below and in particular with respect to the exemplary method shown in Fig. 4, the device 10, which is resident in the working station 40 running as software routines, determines an RP based on the received EEG signal, determines whether patient motion is likely to happen based on the received EEG signal, and provides one or more control signals, if it is predicted that patient motion is likely to happen. The one or more control signals are usable for controlling an apparatus to perform a function to reduce motion artefacts during the medical imaging.

Fig. 3 illustrates an alternative example of a system 100, in accordance with an embodiment of the present disclosure. In this example, the system 100 is a radiation therapy system. The radiation therapy system comprises a device 10, a monitoring system 20, and a radiation therapy delivery apparatus 50, e.g., comprising a linac to generation the radiation beam and a multileaf collimator (MLC) to modulate the beam in accordance with an intensity modulated radiation therapy (IMRT) plan.

In a typical intensity modulated radiation therapy (IMRT) implementation, a beam of high-energy particles (e.g., electrons, photons, protons, ions) is generated by a linear particle accelerator (linac). The target volume (e.g., malignant tumour) of an oncology patient is disposed in the path of the radiation beam. To more precisely irradiate the target volume while limiting radiation exposure of neighbouring organs at risk (OARs), a multi-leaf collimator (MLC) is used to block designed portions of the radiation beam to provide a radiation beam of a desired shape. The MLC segments the radiation beam into "beamlets" that can be turned on or off by pre-programmed movement of leaves of the MLC. For additional dosage control, multiple beam pulses can be applied, with each beam pulse at a chosen energy and beam shape obtained by adjusting the beam energy and suitably configuring the MLC.

In the example of Fig. 3, the EEG signal measured by the monitoring system 20 is provided to the device 10, which is a separate device configured to communicate with the monitoring system 20 through a wireless and/or a wire-based interface. The device 10 determines an RP based on the received EEG signal, determines whether patient motion is likely to happen based on the received EEG signal, and provides one or more control signals, if it is predicted that patient motion is likely to happen. The one or more control signals are usable for controlling an apparatus to assure that radiation dose is delivered according to a planned dose map during the radiation therapy. This will be explained hereafter and particularly with respect to the embodiments shown in Figs. 4 and 5.

Fig. 4 illustrates a flow chart describing a computer-implemented method 200 for monitoring motion during medical imaging or during radiation therapy. Beginning at block 210, i.e. step a), a device, such as device 10 shown in Fig. 1, may receive an EEG signal measured from a patient. The patient may lie on a patient table in a medical imaging system shown in Fig. 2 or in a radiation therapy system shown in Fig. 3.

At block 220, i.e. step b), the device 10 determines an RP based on the received EEG signal.

The brain's current motor activity may be monitored in real time through EEG, which can be further employed for prediction of the next voluntary motor task. Real-time EEG might present novel non-muscular control channel Brain Computer Interfaces (BCIs) for delivering messages and commands to the external world. Studies have shown that EEG comprises enough real-time information to be utilized for different purposes/tasks such as internet browsing, controlling environment (e.g., light, television, and temperature), word processing, controlling a two-dimensional cursor movement on screen, or even operating neuro-prosthesis. EEG data collected prior to imminent movement, which associates with motor preparation and planning period of the brain present substantial prediction potentials.

In humans, spontaneous movements may be preceded by early brain signals. One such signal is the readiness potential (RP) that gradually arises within the last second preceding a movement. Many studies have shown that movements are preceded by early brain signals. A brain-computer interface may be used to detect RP. As will be discussed below, when the signal is detected, a spontaneous movement is expected and the imaging device may react to prevent the movement (veto) or to start measures to counteract the motion pattern to reduce motion artefacts.

The implementation of a motor task in humans measured over the primary motor cortex is preceded by a slow decrease in the EEG amplitude (within at least 500ms) and this potential is known as an MRCP. The MRCP produced in corporation with the planning and execution of a cue-based movement is known as contingent negative variation (CNV), and the one generated in response to self-paced movement is known as readiness potential (RP). The MRCP is present in real as well as in imaginary volitional movements. The MRCP comprises three events called readiness potential (RP) or BP, motor potential, and movement-monitoring potential (MMP), which are thought to reflect movement planning/preparation, execution, and control of performance, correspondingly. BP or RP is a negative cortical potential, which starts to grow around 1.5 to 1 s prior to the onset of a voluntary movement. It has two fundamental segments: the first part is a slow-rising negative segment which develops about 1.5 s before the movement onset, known as "early BP," and is more distinguished in the central-medial scalp, while the second part has a steeper slope and happens around 400―500ms before the movement onset and is called "late BP" which has maximum amplitude over the primary motor cortex.

In some examples, the RP may be determined through known machine learning methods. For example, a machine-learning module may be trained with data from prior patients to derive a correlation between the RP and the EEG signal. Methods for generating such a function may include, but are not limited to, artificial neural networks, Bayesian estimators, support vector machines, and nearest integer classifier.

In some examples, the device 10 may receive one or more physiological signals different from the EEG signal. Examples of such a physiological signal may include, but are not limited to, skin appearance (e.g., colour, texture), temperature, respiration rate, heart rate, perspiration, conductivity, mechanical pressure, and camera-based face expression recognition indicative of unease. The one or more additional physiological signals may be used as a further indicator of upcoming motion events and may be correlated or used as classifiers for the machine-learning module for the RP detection.

At block 230, i.e. step c), the device 10 determines whether patient motion is likely to happen based on the received EEG signal.

In some examples, if the determined RP exceeds a threshold value (e.g., the probability of motion is high), the device may determine that a patient motion is likely to happen. If the determined RP is less than or equal to the threshold value, the device may determine that a motion is not predicted to occur or is insufficient to impede the image reconstruction or is insufficient to impede the radiation dose delivery.

In some examples, if the determined RP is outside of a range of values indicating a tolerated range of motion of the patient, the device may determine that a patient motion is likely to happen. If the determined RP is within the range of values, the device may determine that a motion is not predicted to occur or is insufficient to impede the image reconstruction or is insufficient to impede the radiation dose delivery.

At block 240, i.e. step d), the device provides a control signal via the output unit as output, if it is predicted that patient motion is likely to happen. The control signal is usable for controlling a device to perform a function to reduce motion artefacts during the medical imaging or to assure that radiation dose is delivered according to a planned dose map during the radiation therapy.

Fig. 5 shows an overview 300 of exemplary control signals generated based on the EEG data. As shown in block 310, the device receives an EEG signal, which may be measured by a CBI with an EEG sensor. The device may decode and analyse the EEG by signal processing and a machine-learning module. The machine-learning module has been trained to derive the RP from the EEG signal. The determined RP may be compared with a threshold value or a range of values indicating a tolerated range of motion of the patient to determine whether an upcoming patient motion is likely to happen. One or more control signals may be generated if it is determined that an upcoming patient motion is likely to happen.

Additionally, further vital signs may provide an optional input signal for the motion detection. For example, 3D vital signs, eye motion, and/or a physiological signal shown in block 312 may be an indicator of direct or upcoming motion events and can be correlated or used as classifiers for the machine-learning module.

An interface module may control one or more different interfaces based on the control signal(s) during an imaging sequence or during a radiation therapy process.

In some examples, as shown in block 314, the control signal generated by the device may comprise a signal configured to control an informing device to inform the patient that patient motion is likely to happen such that the patient can proactively react. The interface then delivers the control signal to a corresponding informing device. The patient may be informed via one or more informing devices, such as an optical interface (e.g., LED, Monitor), a mechanical actuator integrated into RF coil or matrass, a haptic feedback device, and an actuator in a glove. Thus, the patient can be actively warned or triggered if an unconscious motion event is upcoming and patient can proactively react.

In some examples, as shown in block 316, the control signal generated by the device may comprise a signal configured to control a robot and/or an actuator to provide a counter-measure such that a counter force is applied to compensate for the patient motion. Actuators may be located in the head/body-fixation holder, in the head coil, and/or in the mattress. In this way, the upcoming patient motion may be prevented mechanically. In these examples, mechanical means, such as immobilization devices or even counteractive actuators, may be used to limit or counteract the motion as it is performed by the patient.

In some examples, as shown in block 318, the control signal generated by the device may comprise a signal configured to control a mechanical control unit of a patient bed, such as movable table 32 shown in Fig. 2. For example, the mechanical control unit of the patient bed may be triggered by a detection of an upcoming patient motion, and a translation or three-dimensional (3D) counter movement can be triggered accordingly.

In some examples, as shown in block 320, the control signal generated by the device may comprise a signal configured to control ambient parameters, such as audio or light, to provide feedback for the patient and the patient can proactively react.

In some examples, the control signal generated by the device may comprise a signal configured to control an imaging device to modify a medical imaging acquisition process in order to account for the patient motion that is likely to happen.

For example, an interface to an MR imaging device may directly change the MRI sequence and system parameters as function of the determined RP parameter.

In a first example, it is possible to adapt the k-space acquisition to account for motion that is about to happen. MR data is acquired in k-space and the motion sensitivity drops with distance from the center of k-space. It is therefore proposed to use the information of motion that is about to happen to change the acquisition protocol such that motion-insensitive parts of k-space are filled.

In a second example, it is also possible to adapt the MR sequence in time domain to account for motion that is about to happen. MR sequences consist of parts in the time domain that are more sensitive to motion than other parts of the same sequence. Examples of motion-sensitive parts are special RF pulses as spatial-spectral pulses, magnetization preparation parts that combine several RF pulses and gradients, diffusion gradient pulses, and/or acquisition windows. The information from the EEG sensor about motion that is about to happen may be used to adapt the timing of the MR sequence in real-time to avoid the play out of such sensitive parts during motion. This approach may be made possible by the fact that the ahead time of the motion warning signal from the CBI with respect of the actual motion is in the order of several 100 ms, which is long compared to the typical length of parts of MR sequences and the time scales that are required by the MR system to adapt/change the MR sequence.

In some examples, as shown in block 324, the control signal generated by the device may comprise a signal to control an x-ray tube, if the imaging device is a CT scanner. For example, the flow of electricity to the X-ray tube may be ceased, when the motion is predicted to occur. For example, the x-ray dose may be reduced when the motion is predicted to occur.

In some examples, as shown in block 326, the control signal generated by the device may comprise a signal configured to control a therapy device to modify a therapy process in order to account for the patient motion that is likely to happen. In some examples, the therapy process may be ceased when the patient motion is predicted to occur. In some examples, the therapy delivery may be redirected. For example, computerized IMRT planning may be performed to determine a program of beam pulses and their beam energies and MLC configurations in order to achieve the desired dose distribution objectives. MLCs used in clinical radiation therapy can modulate hundreds or even thousands of beamlets, and additional adjustments can be made in the number of beam pulses and the beam energy of each pulse. The IMRT plan may be adjusted in response to the detection of an upcoming patient motion to satisfy the plan objectives specified by the patient's doctor.

Optionally, the system 100 may further comprise a motion detector (not shown). When the monitoring system, such as CBI, issues a veto signal to the patient, the patient may or may not be able to suppress the motion. It is important to detect the latter case, because then the MR system should at least prevent the motion mechanically and/or adapt the MR sequence. It is therefore proposed that a respective motion detection is triggered/switched to an active state by the veto signal. This may have a first advantage that false positive motion detection events are prevented at other times. It may have a second advantage that the detection threshold of the motion detector can be chosen relatively low, because in case of a veto signal the probability of motion is relatively high. Only in case of a false positive veto signal issued by the CBI (meaning that there is actually no RP), the probability of motion is zero. Fig. 6 illustrates an exemplary feedback-reaction loop-model 400 for implementing the method 200.

Beginning at block 410, a device, such as device 10 shown in Fig. 1, detects the RP based on the received EEG signal. For example, the RP may be derived from a machine-learning detection platform. At block 420, a threshold value may be applied to the RP to decide whether patient motion is likely to happen and a counter-measure needs to be performed. For example, if the determined RP exceeds a threshold value (e.g., the probability of motion is high), the device may determine that a motion is predicted to occur and proceeds to a step 430. If the determined RP is less than or equal to the threshold value, the device may determine that a motion is not predicted to occur or is insufficient to impede the image reconstruction. In this case, the method will return to the step 410 to continue to detect the RP from the patient. At block 430, a statistical module for sensitivity and latency may be used to provide parameters for a delay. At block 440, one or more control signals are generated to control at least one apparatus to perform one or more counter-measures as discussed in Fig. 5. At block 450, it is determined whether the imaging process is still active. If it is determined the imaging process is still active, the method will return to the step 410 to continue to detect the RP from the patient. If it is determined the imaging process is not active, the method stops.

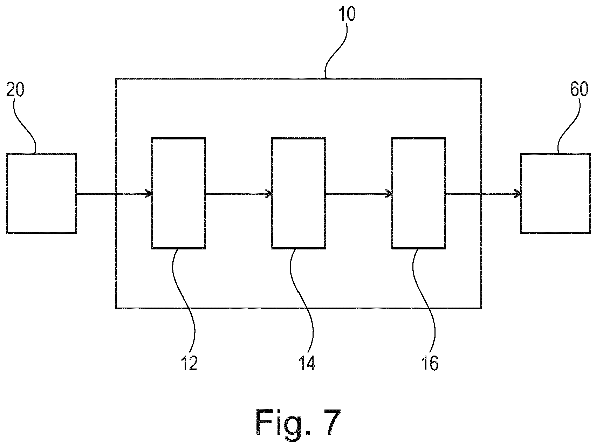

The start of RP regarding the movement onset may vary considerably among different conditions of movement and among patients. Towards this end, Fig. 7 shows an example of a patient learning and guidance tool 500. The patient learning and guidance tool 500 comprise a device 10, a monitoring system 20, and an informing device 60.

The device 10 and the monitoring system 20 may be respectively substantially functionally equivalent to the device 10 and the monitoring system 20 illustrated in Fig. 1,

The informing device 60 may be one or more of an optical interface, a mechanical actuator integrated into RF coils or mattress, a haptic feedback device, and an actuator in a glove. The patient may be trained to be informed about immanent own motion via one or more of the above-mentioned informing devices 60. The patient learning and guidance module is proposed to allow the patient to adapt to reaction. There might be patients, who are not appropriate for EEG prospective motion controller (e.g., sedated or hampered patients). The learning module decides if a patient is able to use the proposed method.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A device (10) for monitoring motion during medical imaging or during radiation therapy, comprising:
- an input unit (12);
- a processing unit (14); and
- an output unit (16);
wherein the input unit is configured to receive an electroencephalogram, EEG, signal measured from a patient;
wherein the processing unit is configured to:
- determine a readiness potential, RP, based on the received EEG signal;
- determine whether patient motion is likely to happen based on the received EEG signal; and
- provide a control signal via the output unit as output, if it is predicted that patient motion is likely to happen; and
wherein the control signal is usable for controlling an apparatus to perform a function to reduce motion artefacts during the medical imaging or to assure that radiation dose is delivered according to a planned dose map during the radiation therapy.

2. Device according to claim 1,
wherein the processing unit is configured to apply a pre-trained machine learning algorithm to derive the RP from the received EEG signal.

3. Device according to claim 1 or 2,
wherein the input unit is configured to receive at least one physiological signal different from the EEG signal; and
wherein the processing unit is configured to determine whether patient motion is likely to happen based on the received EEG signal and the at least one physiological signal.

4. Device according to any one of the preceding claims,
wherein the control signal comprises a signal configured to control an informing device to inform the patient that patient motion is likely to happen such that the patient can proactively react.

5. Device according to any one of the preceding claims,
wherein the control signal comprises a signal configured to control a robot and/or an actuator to provide a counter-measure such that a counter force is applied to compensate for the patient motion.

6. Device according to claim 5,
wherein the actuator is located in one or more of the followings:
- in a head-fixation holder;
- in a body-fixation holder;
- in a head coil; and
- in a mattress.

7. Device according to any one of the preceding claims,
wherein the control signal comprises a signal configured to control an imaging device to modify a medical imaging acquisition process in order to account for the patient motion that is likely to happen.

8. Device according to any one of the preceding claims,
wherein the control signal comprises a signal configured to control a therapy device to modify a therapy process in order to account for the patient motion that is likely to happen; and
wherein the modification of the therapy process comprises one of:
- ceasing the therapy process; and
- redirecting therapy delivery.

9. Device according to any one of the preceding claims,
wherein the control signal comprises a signal configured to activate a motion detector to detect whether the patient is moving.

10. A system (100), comprising:
- a monitoring system (20) configured to measuring an electroencephalogram, EEG, signal of a patient; and
- a device (10) according to any one of preceding claims configured to receive the measured EEG signal and to provide a control signal as output, if it is predicted that patient motion is likely to happen.

11. System according to claim 10, further comprising one or more of:
- an informing device configured to inform the patient that patient motion is likely to happen, in response to the control signal;
- a robot and/or an actuator to provide a counter-measure such that a counter force is applied to compensate for the patient motion in response to the control signal; and
- a motion detector configured to be activated to detect whether the patient is moving, in response to the control signal.

12. System according to claim 10 or 11,
wherein the system is a medical imaging system that comprises an imaging device configured to acquire image data of the patient; and
wherein the imaging device is configured to modify a medical imaging acquisition process in order to account for the patient motion that is likely to happen, in response to the control signal.

13. System according to claim 10 or 11,
wherein the system is a radiation therapy system comprising a therapy device configured to deliver ionizing radiation; and
wherein the therapy device is configured to modify a therapy process in order to account for the patient motion that is likely to happen, in response to the control signal.

14. A computer-implemented method (200) for monitoring motion during medical imaging or during radiation therapy, comprising:
a) receiving (210) an electroencephalogram, EEG, signal measured from a patient;
b) determining (220) a readiness potential, RP, based on the received EEG signal;
c) determining (230) whether patient motion is likely to happen based on the received EEG signal; and
d) providing (240) a control signal via the output unit as output, if it is predicted that patient motion is likely to happen;
wherein the control signal is usable for controlling an apparatus to perform a function to reduce motion artefacts during the medical imaging or to assure that radiation dose is delivered according to a planned dose map during the radiation therapy.

15. A computer program product comprising instructions which, when the program is executed by at least one processing unit, cause the at least one processing unit to carry out the steps of the method according to claim 14.
